# FASCICULE DE BREVET EUROPEEN

(11) **EP 0 733 902 B1**
(45) Date de publication et mention de la délivrance du brevet: **31.10.2001**
(21) Numéro de dépôt: 96400587.0
(22) Date de dépôt: 21.03.1996
(51) Int. Cl.: G01N 33/44, G01N 27/22

(54) **Procédé et dispositif de suivi de polymérisation de systèmes réactifs**
Verfahren und Vorrichtung zur Überwachung der Polymerisation von reaktiven Systemen
Method and apparatus for monitoring the polymerization of reactive systems

(30) Priorité: 23.03.1995 FR 9503399
(43) Date de publication de la demande: 25.09.1996
(73) Titulaire: Snecma Moteurs, 75015 Paris (FR)
(72) Inventeur: Boiteux, Gisèle Antoinette Françoise, 69270 - Fontaines/Saone (FR); Stephan, Françoise Antoinette Marie, 29890 - Kerlouan (FR); Chatellier, Jean-Yves François Roger, 94110 - Arcueil (FR); Ulanski, Jacek, 90735 - Lodz (PL); Seytre, Gérard Paul Claude, 69380 - Dommartin (FR)
(74) Mandataire: Berrou, Paul

(56) Documents cités:
- EP-A- 0 516 367
- FR-A- 2 645 275
- US-A- 4 399 100
- US-A- 5 032 525
- INSTRUMENTATION SCIENCE & TECHNOLOGY, vol. 22, no. 3, Août 1994, WAMPLER US, pages 259-271, XP000469077 B. TWOMBLY, ET AL. : "SIMULTANEOUS DYNAMIC MECHANICAL ANALYSIS AND DIELECTRIC ANALYSIS OF POLYMERS (DMA-DEA)"
- PATENT ABSTRACTS OF JAPAN vol. 9, no. 72 (P-345), 2 Avril 1985 & JP-A-59 204749 (DAIAFOIL KK), 20 Novembre 1984,

## Description

L'invention concerne un procédé et un dispositif de suivi de polymérisation de systèmes réactifs. Elle s'applique en particulier au suivi et au contrôle de la polymérisation des matériaux composites en résine.

Les matériaux composites comportent généralement un matériau de base, tel que par exemple une résine, qui est renforcé par des fibres. Les fibres peuvent être en divers matériaux tels que par exemple le carbone, le verre, le graphite ou le nylon. La fabrication de ces matériaux composites comporte une étape finale de durcissement à hautes températures dans un autoclave. Pendant cette étape de durcissement, le matériau composite passe par différents stades auxquels correspondent des propriétés différentes du matériau. A basses températures et pressions, les solvants et l'eau sont éliminés par cuisson et une imidation de la résine se produit. Lorsque la température augmente, la résine passe par une transition vitreuse puis lors d'un stade final, le matériau est chauffé sous haute pression et passe par une réaction de réticulation.

Les qualités de la résine pouvant varier d'un lot à l'autre, pour maîtriser les propriétés des matériaux composites il est nécessaire, au cours de chaque processus individuel de durcissement, de pouvoir adapter le cycle de cuisson et le moment d'application d'une pression en fonction du stade de polymérisation du matériau.
Pour cela, il est nécessaire de pouvoir disposer d'une méthode de suivi de polymérisation de la résine, ainsi que d'une commande adaptative de température et d'une commande adaptative de pression qui permettent de piloter l'autoclave en cours de cuisson du matériau en fonction de l'état de polymérisation du matériau.

Le document "Instrumentation Science et Technology, 22(3), 259-271 (1994)" décrit une méthode de suivi de polymérisation d'une résine au cours de la cuisson d'un composite qui consiste à placer un capteur de température et un capteur diélectrique dans le matériau à surveiller, le capteur diélectrique étant associé à un microdiélectromètre, à exciter le capteur diélectrique avec un signal sinusoïdal délivré par une source de courant alternatif, à détecter la réponse du matériau à cette excitation et à traiter les signaux obtenus pour obtenir la permittivité diélectrique et le facteur de pertes diélectriques du matériau.
Le brevet US-A-5 032 525 décrit un dispositif automatique permettant de piloter un autoclave en fonction de l'état de polymérisation d'un matériau composite.
Le brevet US 4 399 100 décrit un procédé de contrôle automatique de la cuisson d'un matériau polymère.
Les dispositifs décrits dans ces différents documents utilisent tous un appareillage de coût élevé, en particulier le coût du diélectromètre est prohibitif, et par ailleurs le traitement des signaux obtenus est très complexe.
En outre, les méthodes qu'ils décrivent ne permettent pas, dans la réponse du matériau à l'excitation, d'isoler la contribution des ions et la contribution des dipôles ce qui rend l'identification des phénomènes très difficile.

Le but de l'invention est de réaliser un procédé et un dispositif de suivi de polymérisation de mélanges réactifs qui soient moins coûteux, qui permettent d'isoler, dans la réponse du matériau à une excitation, les contributions ioniques et dipolaires et qui permettent ainsi d'identifier, pendant le cycle de cuisson, les différents phénomènes qui interviennent et l'état de polymérisation du matériau. La connaissance de l'état de polymérisation du matériau peut alors être utilisée pour piloter l'autoclave automatiquement sans faire intervenir un opérateur.
Pour cela, le procédé selon l'invention consiste pendant la durée du cycle de cuisson, à appliquer au matériau des cycles de tension, chaque cycle de tension comportant au moins une phase de polarisation pendant laquelle un capteur microdiélectrométrique placé dans le matériau à polymériser est excité par un échelon de tension continue et au moins une phase de dépolarisation pendant laquelle la tension est interrompue et en ce qu'il consiste à mesurer, pendant la phase de polarisation, l'évolution du courant électrique induit dans le matériau par l'échelon de tension continue. Le traitement des valeurs mesurées permet d'extraire des paramètres caractéristiques de l'état d'avancement de la polymérisation du matériau et de déterminer automatiquement les valeurs de température et de pression à appliquer à l'autoclave pour la poursuite du processus de polymérisation.

Selon l'invention, le procédé de suivi de polymérisation de systèmes réactifs dans lequel un matériau à polymériser est durci par cuisson selon un cycle de température et de pression évolutif en fonction du temps, caractérisé en ce qu'il consiste, pendant le processus de cuisson, à appliquer au matériau des cycles de tension, chaque cycle de tension comportant au moins une phase de polarisation pendant laquelle un échelon de tension continue est appliqué au matériau et au moins une phase de dépolarisation pendant laquelle la tension est interrompue, et en ce qu'il consiste, pendant chaque cycle de tension :
- à mesurer la température de cuisson,
- à détecter et mesurer des variations d'un courant électrique induit dans le matériau par l'échelon de tension continue pendant la phase de polarisation,
- à enregistrer et traiter les valeurs du courant et de la température mesurées de manière à extraire des paramètres caractéristiques de l'état d'avancement de la polymérisation du matériau,
- à déterminer les valeurs de température et de pression à appliquer au matériau en fonction de l'état d'avancement de la polymérisation du matériau.

L'invention concerne également un dispositif de suivi de polymérisation de systèmes réactifs dans lequel un matériau à polymériser est durci par cuisson selon un cycle de température et de pression évolutif en fonction du temps, caractérisé en ce qu'il comporte un four programmable, au moins un capteur microdiélectrométrique destiné à être disposé dans le matériau à polymériser, au moins un capteur de température, un électromètre comportant une source de tension destiné à délivrer une tension continue et comportant des moyens de mesure d'un courant électrique dans le matériau, un micro-ordinateur destiné à gérer l'acquisition, le stockage et le traitement des informations provenant des capteurs et à délivrer en retour des valeurs de consigne de pression et de température permettant de contrôler le cycle de polymérisation.

D'autres particularités et avantages de l'invention apparaîtront clairement dans la suite de la description donnée à titre d'exemple non limitatif et faite en regard des figures annexées qui représentent :
- la figure 1, un schéma synoptique du dispositif de contrôle du cycle de cuisson d'un matériau composite, selon l'invention ;
- la figure 2, un schéma synoptique du procédé de suivi de la polymérisation d'un système réactif, selon l'invention ;
- les figures 3a et 3b, un exemple de cycle de tension appliqué au matériau en fonction du temps et respectivement, un exemple correspondant de réponse en courant, selon l'invention
- la figure 4, l'évolution du courant de conduction en fonction de la température au cours de la polymérisation d'une résine, selon l'invention ;
- la figure 5, l'évolution du courant d'absorption en fonction du temps pendant une phase de polarisation, selon l'invention ;
- les figures 6a et 6b, respectivement les courbes d'évolution du paramètre A2 et de la pente n2 en fonction de la température, selon l'invention.

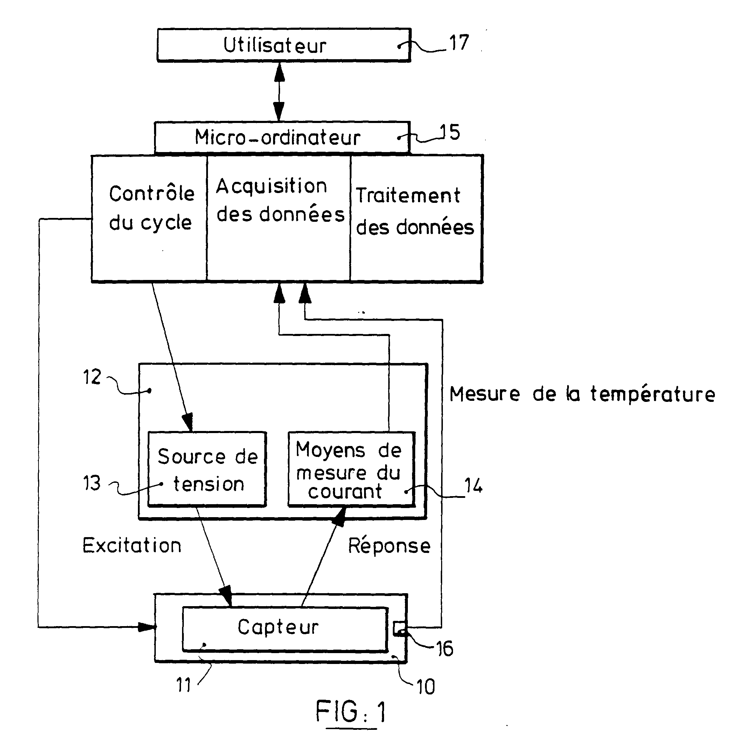

Le dispositif de contrôle du cycle de cuisson d'un matériau composite représenté selon la figure 1 comporte un four programmable 10 ou un autoclave ; un capteur microdiélectrométrique 11 destiné à être disposé dans un matériau devant subir un cycle de polymérisation à l'intérieur de l'autoclave 10 ; un capteur de température 16, par exemple un thermocouple, destiné à détecter la température à l'intérieur de l'autoclave ; un électromètre 12 à source de tension 13 incorporée destiné à délivrer une tension continue d'excitation du matériau en cours de polymérisation et comportant des moyens de mesure 14 de la réponse en courant du matériau ; un micro-ordinateur 15 destiné à gérer l'acquisition, le stockage et le traitement des informations provenant du capteur microdiélectrométrique 11 et du capteur de température 16, et à délivrer en retour des valeurs de consigne de pression et de température permettant de contrôler le cycle de polymérisation. Des informations peuvent également être transmises manuellement au micro-ordinateur 15 par l'utilisateur 17.
Le capteur microdiélectrométrique 11 est par exemple du type comportant deux électrodes disposées en peignes interdigités.

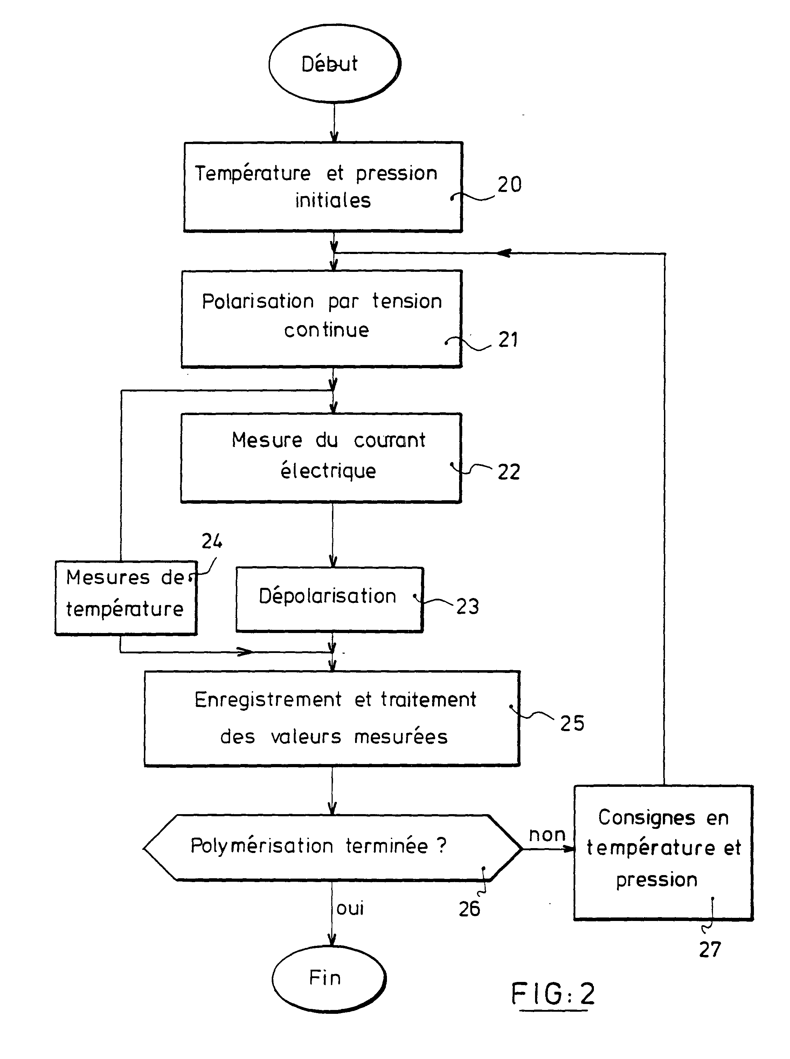

Le fonctionnement du dispositif est le suivant. Le cycle de cuisson est contrôlé automatiquement par le micro-ordinateur 15 qui délivre des valeurs de consignes en température et en pression appliquées en entrée du four programmable 10. Les valeurs de consignes sont déterminées à partir des mesures effectuées par le capteur de température 16 et le capteur microdiélectrométrique 11. Ces mesures sont effectuées pendant toute la durée du cycle de cuisson de la façon décrite ci-après en référence à la figure 2 qui représente un schéma synoptique du procédé de suivi de la polymérisation d'un système réactif, selon l'invention.
Le cycle de cuisson est initialisé, dans une étape 20, par l'utilisateur 17, en imposant des valeurs de température et de pression initiales. A partir de ce moment et pendant toute la durée du cycle de cuisson, des cycles de tension successifs sont appliqués au capteur microdiélectrométrique 11. Chaque cycle de tension comporte au moins une phase de polarisation et une phase de dépolarisation du matériau. Pendant la phase de polarisation du matériau, dans l'étape 21, le capteur microdiélectrométrique 11 est excité par un échelon de tension continue, cette tension continue étant appliquée sur l'une des électrodes du capteur 11 pendant une durée d1 prédéterminée appelée temps de polarisation du matériau. En réponse à l'excitation en tension et pendant la durée d1 d'application de cette tension continue, un courant électrique s'établit dans le matériau et est capté par la deuxième électrode du capteur 11.

Des mesures de ce courant électrique sont alors effectuées, dans une étape 22, par l'électromètre 12, à différents instants pendant la durée d'application de la tension continue de polarisation. Au début de l'application de la tension continue, le courant électrique appelé courant d'absorption, décroît rapidement puis se stabilise à une valeur statique appelée courant statique ou courant de conduction.

Pendant la phase de dépolarisation, dans l'étape 23, la tension de polarisation est interrompue pendant une durée d2 prédéterminée appelée temps de dépolarisation du matériau, la durée d2 étant nettement supérieure, par exemple dix fois supérieure, à la durée dl. Pendant chaque cycle de tension, une ou plusieurs mesures de température sont effectuées dans une étape 24.

Dans une étape 25, les valeurs du courant mesurées pendant la phase de polarisation du matériau et les valeurs de température mesurées pendant le cycle de tension sont enregistrées et traitées dans le micro-ordinateur 15 de manière à extraire des paramètres caractéristiques de l'état d'avancement de la polymérisation du matériau. Un test permettant de déterminer la fin de la polymérisation est effectué dans une étape 26. Si ce test est positif, le cycle de cuisson est terminé, si ce test est négatif, dans une étape 27, le micro-ordinateur décide automatiquement de modifier ou non les valeurs de température et de pression à appliquer au matériau. Cette décision est transmise au four programmable pour exécution et un nouveau cycle de tension est effectué.

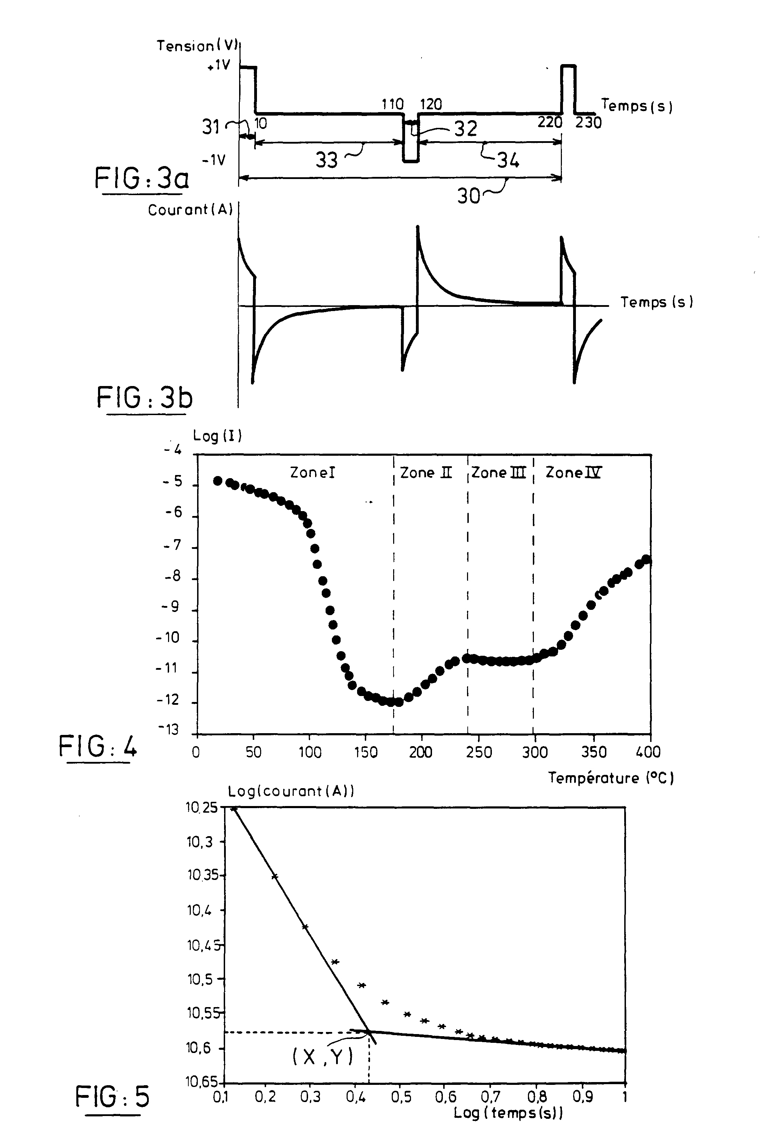

Les figures 3a et 3b représentent respectivement un exemple de cycle de tension appliqué au matériau en fonction du temps et un exemple correspondant de réponse en courant.

Le cycle de tension 30 comporte deux phases de polarisation 31, 32, et deux phases de dépolarisation 33, 34. Pendant les deux phases de polarisation 31,32, le capteur microdiélectrométrique 11 est excité par un échelon de tension continue respectivement positive et négative appliqué sur l'électrode excitatrice du capteur 11. L'échelon de tension continue est appliqué pendant une durée prédéterminée d1 nécessaire à l'établissement dans le matériau d'un courant de conduction stabilisé à une valeur statique.

Chaque phase de polarisation est suivie d'une phase de dépolarisation pendant laquelle l'électrode excitatrice du capteur microdiélectrométrique 11 est mise en court circuit.

Dans l'exemple représenté sur les figures 3a et 3b, la tension continue appliquée pendant les phases de polarisation 31, 32 a une amplitude de 1 volt, les temps de polarisation et de dépolarisation sont respectivement de 10 secondes et de 100 secondes. Ces valeurs sont fortement dépendantes de la nature du matériau composite en cours de polymérisation.
La réponse en courant du matériau dépend non seulement de la valeur de l'échelon de tension appliqué mais également de ceux imposés lors des cycles de tension précédents.
Pour que les grandeurs mesurées soient caractéristiques de l'état du matériau à un instant précis du cycle de polymérisation, il est nécessaire de s'assurer que les phénomènes transitoires résultant des cycles précédents soient supprimés. Cette condition est satisfaite en imposant un temps de dépolarisation nettement supérieur, par exemple dix fois supérieur, au temps de dépolarisation.

L'application d'un échelon de tension continue alternativement positive et négative permet d'atténuer les phénomènes de polarisation des électrodes.

La figure 4 représente l'évolution du courant de conduction en fonction de la température au cours de la polymérisation d'une résine connue sous le nom de résine PMR15.

L'évolution du courant de conduction représentée sur cette figure 4 correspond au cas d'une polarisation par une tension continue alternativement positive et négative d'amplitude un Volt pour un traitement thermique constitué d'une rampe de un degré Celsius par minute. Les variations du courant de conduction en fonction de la température sont représentées en coordonnées semi-logarithmiques.

Le courant de conduction est mesuré à la fin de la phase de polarisation quant le courant est stabilisé à une valeur statique. Dans le cas présent la mesure du courant de conduction est effectuée quand le temps de polarisation est égal à 10 secondes.

La courbe d'évolution du courant de conduction permet de mettre en évidence plusieurs zones de température caractéristiques de la polymérisation de la résine PMR15.

La zone I correspond à la réaction d'imidation qui se caractérise par une décroissance du courant de conduction.

La zone II correspond au passage de la transition vitreuse du prépolymère qui se caractérise par une augmentation du courant de conduction.

La zone III correspond au début de la formation du réseau tridimensionnel qui se caractérise par une décroissance du courant de conduction.

La zone IV, au delà de 300°C correspond au passage de la transition vitreuse de la matrice PMR15 réticulée qui se caractérise par une augmentation du courant de conduction.

La figure 5 représente l'évolution du courant d'absorption en fonction du temps pendant une phase de polarisation donnée.

Les variations du courant d'absorption sont représentées en coordonnées bilogarithmiques.
Le courant d'absorption est mesuré à différents instants pendant la phase de polarisation au début de l'application de la tension continue, par exemple pendant les cinq premières secondes.

la figure 5 montre que le courant d'absorption décroît très vite avec une pente n1 pendant les premiers instants d'application de la tension de polarisation puis décroît beaucoup plus lentement avec une pente n2 en tendant vers une valeur statique lorsque les instants de mesure correspondent à un temps de polarisation plus long.

Le procédé, selon l'invention, consiste alors à faire intervenir deux lois de puissance empiriques correspondant à la courbe d'évolution du courant d'absorption pendant les premiers instants d'application de la tension de polarisation, respectivement pendant les instants correspondant à un temps de polarisation plus long.

Les deux lois de puissance empiriques sont de la forme :
I1 = A1 t exp (-n1) et I2 = A2 t exp (-n2)
où I1, I2, n1, n2 représentent les courants et les pentes de décroissance du courant d'absorption pour des temps de polarisation respectivement courts et longs, où t représente le temps, et où A1 et A2 sont des paramètres qui ne dépendent pas du temps.

Ces deux lois de puissance sont représentées sur la figure 5 en coordonnées bilogarithmiques. Ce sont deux droites tangentes à la courbe d'évolution du courant d'absorption en fonction du temps d'application de la tension de polarisation respectivement pour les temps courts et longs. Ces deux droites comportent un point d'intersection de coordonnées notées (X, Y).

Les valeurs des paramètres A1, A2, n1, n2, X, Y sont alors déterminées pour chaque phase de polarisation et leurs évolutions en fonction de la température sont étudiées pour caractériser la polymérisation du matériau, constitué de la résine PMR15 dans l'exemple choisi.

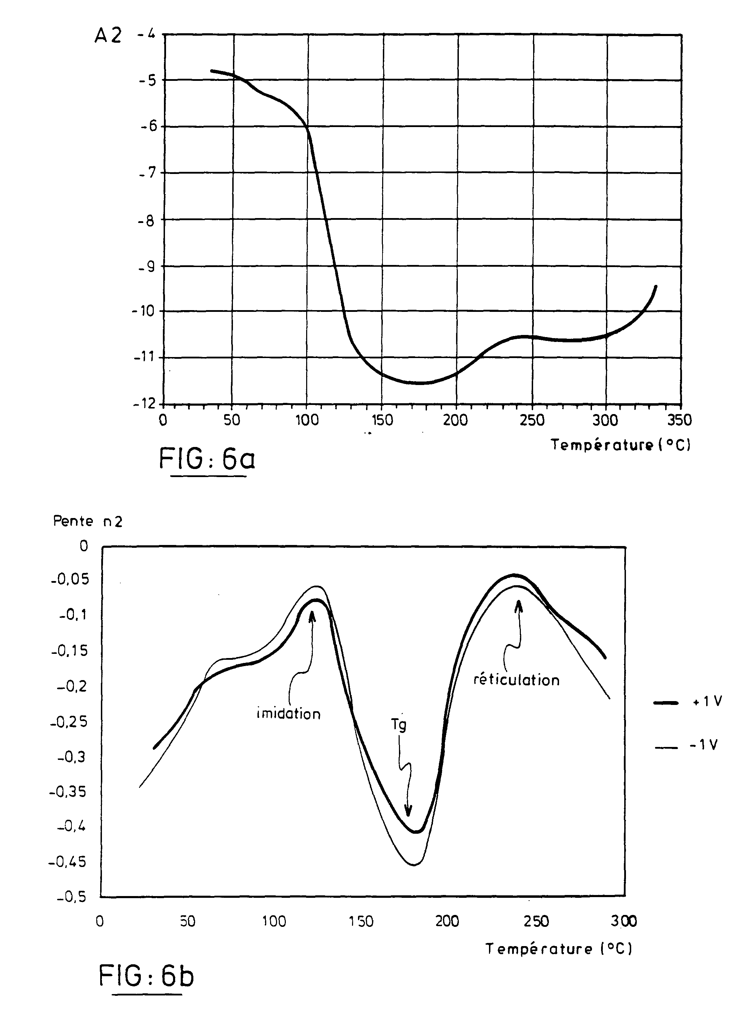

Les figures 6a et 6b, représentent respectivement les courbes d'évolution du paramètre A2 et de la pente n2 en fonction de la température.

Les courbes d'évolution du paramètre A1 et des coordonnées X et Y du point d'intersection des deux lois de puissance ne sont pas représentées car elles sont similaires à la courbe d'évolution du paramètre A2. De la même façon, la courbe d'évolution de la pente n1 est similaire à celle de la pente n2.

La figure 6a montre que l'évolution du paramètre A2 est similaire à l'évolution du courant de conduction en fonction de la température. Il en est également de même pour le paramètre A1 et les coordonnées X et Y. Les grandeurs A1, A2, X, Y sont donc liées à la mobilité des ions et à leur concentration. Leur évolution en fonction de la température permet de mettre en évidence plusieurs zones de température caractéristiques de la polymérisation du matériau, ces zones correspondant à celles déterminées à partir de la courbe d'évolution du courant de conduction.

La courbe d'évolution de la pente n2 met en évidence les trois transitions caractéristiques de la réaction de polymérisation, ces trois transitions étant l'imidation, la transition vitreuse à la température Tg, et la réaction de réticulation. Il en est de même pour la pente ni dont la courbe d'évolution n'est pas représentée. Les pentes n1 et n2 sont représentatives de la mobilité des ions. Elles permettent de déterminer l'état d'avancement de la polymérisation avec une grande sensibilité.

L'invention n'est pas limitée à l'exemple précisément décrit et peut être appliquée à d'autres mélanges réactifs tels que l'époxy, l'époxy renforcé, le polyester, le polyuréthane,.... Le cycle de tension continue appliqué peut être différent, l'amplitude de la tension appliquée et les temps de polarisation et de dépolarisation dépendant fortement de la nature du matériau étudié.
Par ailleurs, le dispositif peut comporter plusieurs capteurs microdiélectrométriques et plusieurs capteurs de température.

## Revendications

1. Procédé de suivi de polymérisation d'un matériau dans lequel le matériau à polymériser est durci par cuisson selon un cycle de température et de pression évolutif en fonction du temps, **caractérisé en ce qu'**il consiste, pendant le processus de cuisson, à appliquer au matériau des cycles de tension, chaque cycle de tension comportant au moins une phase de polarisation pendant laquelle un échelon de tension continue est appliqué au matériau et au moins une phase de dépolarisation pendant laquelle la tension est interrompue, et **en ce qu'**il consiste, pendant chaque cycle de tension :
- à mesurer la température de cuisson,
- à détecter et mesurer des variations d'un courant électrique induit dans le matériau par l'échelon de tension continue, pendant la phase de polarisation,
- à enregistrer et traiter les valeurs du courant et de la température mesurées de manière à extraire des paramètres caractéristiques de l'état d'avancement de la polymérisation du matériau,
- à déterminer les valeurs de température et de pression à appliquer au matériau en fonction de l'état d'avancement de la polymérisation du matériau.

2. Procédé selon la revendication 1, **caractérisé en ce que** l'échelon de tension continue est appliqué pendant une durée prédéterminée nécessaire à l'établissement dans le matériau d'un courant stabilisé à une valeur statique appelée courant de conduction.

3. Procédé selon la revendication 2, **caractérisé en ce que** des paramètres caractéristiques de l'état d'avancement de la polymérisation sont obtenus à chaque cycle de tension, à partir d'une courbe d'évolution du courant électrique en fonction du temps d'application de la tension de polarisation avant la stabilisation du courant à une valeur statique, et en faisant intervenir deux lois de puissance empiriques correspondant à la courbe d'évolution du courant respectivement pour des temps de polarisation courts et longs.

4. Procédé selon la revendication 3, **caractérisé en ce que** la durée de la phase de dépolarisation est de l'ordre de dix fois supérieure à la durée de la phase de polarisation.

5. Procédé selon la revendication 4, **caractérisé en ce que** chaque cycle de tension comporte deux phases de polarisation (31, 32) pendant lesquelles le matériau est polarisé par un échelon de tension continue respectivement positive et négative, chaque phase de polarisation étant suivie d'une phase de dépolarisation (33, 34).

6. Dispositif de suivi de polymérisation d'un matériau composite, **caractérisé en ce qu'**il comporte ; un four programmable (10), au moins un capteur microdiélectrométrique (11) destiné à être disposé dans le matériau à polymériser, au moins un capteur de température (16),un électromètre (12) comportant une source de tension (13) destiné à délivrer une tension continue et comportant des moyens de mesure (14) d'un courant électrique dans le matériau, un micro-ordinateur (15) destiné à gérer l'acquisition, le stockage et le traitement des informations provenant des capteurs (11,16) et à délivrer en retour des valeurs de consigne de pression et de température permettant de contrôler le cycle de polymérisation.

## Patentansprüche

1. Verfahren zur Überwachung der Polymerisation eines Werkstoffs, bei dem der zu polymerisierende Werkstoff durch Brennen in einem sich zeitabhängig entwickelnden Temperatur- und Druckzyklus gehärtet wird,
**dadurch gekennzeichnet,**
**dass** es darin besteht, während des Brennvorgangs zyklisch Spannung an den Werkstoff anzulegen, wobei jeder Spannungszyklus mindestens eine Polarisationsphase enthält, bei der eine Gleichspannungsstufe an den Werkstoff angelegt wird, und mindestens eine Depolarisationsphase enthält, bei der die Spannung unterbrochen wird, und dass es darin besteht, während jedes Spannungszyklus
- die Brenntemperatur zu messen,
- während der Polarisationsphase Schwankungen eines elektrischen Stroms zu erkennen und zu messen, der in dem Werkstoff durch die Gleichspannungsstufe induziert wird,
- die gemessenen Strom- und Temperaturwerte aufzuzeichnen und zu verarbeiten, so dass charakteristische Parameter des Fortschritts der Polymerisation des Werkstoffs gewonnen werden,
- die Werte der auf den Werkstoff auszuübenden Temperatur und des Drucks in Abhängigkeit vom Fortschritt der Polymerisation des Werkstoffs zu bestimmen.

2. Verfahren nach Anspruch 1,
**dadurch gekennzeichnet,**
**dass** die Gleichspannungsstufe an den Werkstoff während einer vorbestimmten Dauer angelegt wird, die erforderlich ist, um in dem Werkstoff einen Strom aufzubauen, der auf einen statischen Wert stabilisiert ist, welcher Leitungsstrom genannt wird.

3. Verfahren nach Anspruch 2,
**dadurch gekennzeichnet,**
**dass** charakteristische Parameter des Fortschritts der Polymerisation bei jedem Spannungszyklus aus einer Entwicklungskurve des elektrischen Stroms in Abhängigkeit von der Anwendung der Polarisationsspannung vor der Stabilisierung des Stroms auf einen statischen Wert sowie durch Anwendung von zwei empirischen Potenzgesetzen entsprechend der Entwicklungskurve des Stroms bei kurzen bzw. bei langen Polarisationszeiten gewonnen werden.

4. Verfahren nach Anspruch 3,
**dadurch gekennzeichnet,**
**dass** die Dauer der Depolarisationsphase ca. das Zehnfache der Dauer der Polarisationsphase beträgt.

5. Verfahren nach Anspruch 4,
**dadurch gekennzeichnet,**
**dass** jeder Spannungszyklus zwei Polarisationsphasen (31, 32) enthält, bei denen der Werkstoff durch eine Stufe mit positiver bzw. negativer Gleichspannung polarisiert wird, wobei auf jede Polarisationsphase eine Depolarisationsphase (33, 34) folgt.

6. Vorrichtung zur Überwachung der Polymerisation eines Verbund-Werkstoffs,
**dadurch gekennzeichnet,**
**dass** sie aufweist:
- einen programmierbaren Ofen (10), mindestens einen mikrodielektrometrischen Sensor (11), der dazu vorgesehen ist, in dem zu polymerisierenden Werkstoff angeordnet zu werden,
- mindestens einen Temperaturfühler (16),
- ein Elektrometer (12) mit einer Spannungsquelle (13), das dazu vorgesehen ist, eine Gleichspannung abzugeben, und mit Messmitteln (14) für einen elektrischen Strom in dem Werkstoff,
- einen Mikrocomputer (15), der dazu vorgesehen ist, die Erfassung, die Speicherung und die Verarbeitung der von den Sensoren (11, 16) kommenden Daten zu verwalten und Solldruck- und Solltemperaturwerte abzugeben, mittels derer der Polymerisationszyklus überwacht werden kann.

## Claims

1. Method of monitoring the cure of a material, in which the material to be cured is cured by curing it according to a temperature/pressure cycle which changes with time, **characterized in that** it consists, during the curing process, in applying voltage cycles to the material, each voltage cycle comprising at least one polarization phase during which a DC voltage step is applied to the material and at least one depolarization phase during which the voltage is interrupted, and **in that** it consists, during each voltage cycle:
- in measuring the cure temperature;
- in detecting and measuring variations in an electric current induced in the material during the polarization phase by the DC voltage step;
- in recording and processing the current and temperature values measured so as to extract parameters characteristic of the state of cure of the material; and
- in determining the temperature and pressure values to be applied to the material depending on the state of cure of the material.

2. Method according to Claim 1, **characterized in that** the DC voltage step is applied for a predetermined time necessary for a steady current, called the conduction current, to be established in the material.

3. Method according to Claim 2, **characterized in that** the parameters characteristic of the state of cure are obtained, at each voltage cycle, from a curve of variation of the electric current as a function of the time during which the polarization voltage is applied before the current becomes steady, and by making use of two empirical power laws corresponding to the curve of variation of the current for a short polarization time and a long polarization time, respectively.

4. Method according to Claim 3, **characterized in that** the duration of the depolarization phase is of the order of ten times longer than the duration of the polarization phase.

5. Method according to Claim 4, **characterized in that** each voltage cycle comprises two polarization phases (31, 32) during which the material is polarized by a positive and a negative DC voltage step, respectively, each polarization phase being followed by a depolarization phase (33, 34).

6. Device for monitoring the cure of a composite material, **characterized in that** it comprises, a programmable oven (10), at least one micro-dielectrometer sensor (11), intended to be placed in the material to be cured, at least one temperature sensor (16), an electrometer (12), which includes a voltage source (13) intended to deliver a DC voltage and means (14) for measuring an electric current in the material, and a microcomputer (15) intended to manage the acquisition, storage and processing of the information output by the sensors (11, 16) and, in return, to deliver pressure and temperature setpoint values allowing the cure cycle to be controlled.
